# FASCICULE DE BREVET EUROPEEN

(11) **EP 1 131 113 B1**
(45) Date de publication et mention de la délivrance du brevet: **20.07.2005**
(21) Numéro de dépôt: 99972115.2
(22) Date de dépôt: 15.11.1999
(51) Int. Cl.: A61L 31/10, A61L 31/16

(54) **PROTHESES BIOACTIVES EN MATERIAU CONDUCTEUR REVETU D'UN POLYMERE ET D'UNE SUBSTANCE A PROPRIETES IMMUNOSUPPRESSIVES, ANTISTENOSE ET ANTITHROMBOSE**
BIOAKTIVE PROTHESEN AUS LEITENDEM MATERIAL BESCHICHTET MIT EINEM POLYMER UND EINER SUBSTANZ MIT IMMUNSUPPRESSIVEN , ANTISTENOSEN UND ANTITHROMBOSEN EIGENSCHAFTEN
BIOACTIVE PROSTHESES OF A CONDUCTIVE MATERIAL COATED WITH A POLYMER AND A SUBSTANCE WITH IMMUNOSUPPRESSIVE, ANTISTENOTIC AND ANTITHROMBOTIC PROPERTIES

(30) Priorité: 16.11.1998 FR 9814351
(43) Date de publication de la demande: 12.09.2001
(73) Titulaire: COMMISSARIAT A L'ENERGIE ATOMIQUE, 75015 Paris Cédex 15 (FR); INSTITUT NATIONAL DE LA SANTE ET DE LA RECHERCHE MEDICALE (INSERM), 75654 Paris Cédex 13 (FR); Kerckhoff-Klinik GmbH, 61231 Bad Nauheim (DE)
(72) Inventeur: LE MOEL, Alain, F-92370 Chaville (FR); BETZ, Natacha, F-92100 Boulogne-Billancourt (FR); BUREAU, Christophe, F-91940 Les Ulis (FR); DENIAU, Guy, F-78610 Auffargis (FR); BAQUEY, Charles, F-33160 Le Haillan (FR); DELERIS, Gérard, F-33000 Bordeaux (FR); HABERBOSCH, Werner, D-35398 Giessen (DE)
(74) Mandataire: Audier, Philippe André
(86) Numéro de dépôt international: PCT/FR1999/002795
(87) Numéro de publication internationale: WO 2000/029043

(56) Documents cités:
- EP-A- 0 596 615
- EP-A- 0 873 732
- EP-A- 0 940 144
- FR-A- 2 187 849
- US-A- 4 879 135
- DEGERT C., DUPUY B., LABAT B. AND BAQUEY CH.: "Association of Polyacrylamide beads to polyethylene terephtalate prostheses" BIOMAT., ART. CELLS & IMMOB. BIOTECH., vol. 21, no. 4, 1993, pages 553-561, XP002111293 cité dans la demande
- BAQUEY CH., BEZIADE A., DUCASSOU D. ET BLANQUET P.: "Interet du Greffage Radiochimique de Monomères Vinyliques pour améliorer l' hémocompatibilité des Matériaux artificiels" INNOV. TECH. BIOL. MED., vol. 2, no. 4, 1981, pages 378-389, XP002111294 cité dans la demande

## Description

### Domaine technique

La présente invention concerne des prothèses bioactives présentant notamment des propriétés immunosuppressives, antisténose et antithrombotiques.

De telles prothèses peuvent être utilisées dans le domaine de la cardiologie et de la chirurgie vasculaire comme substitut d'artères ou comme endocalibreurs ou « stents » disposés dans des artères, en particulier dans les artères coronaires, pour éviter tout risque de thrombose ou de resténose.

Des prothèses bioactives peuvent également être utilisées dans de nombreux autres domaines où il est important de conférer à la prothèse une propriété supplémentaire due à la présence d'une substance bioactive.

### Etat de la technique antérieure

Dans le domaine de la cardiologie, il est habituel de traiter les sténoses par angioplastie, c'est-à-dire en introduisant dans l'artère concernée un ballonnet que l'on gonfle sous forte pression au contact du rétrécissement pour le faire disparaître. A la suite de l'angioplastie, on peut disposer dans l'artère concernée un endocalibreur ou « stent » qui empêche que se produise à nouveau une sténose. Toutefois, une complication associée à l'utilisation des ces endocalibreurs provient de la croissance de néo-tissus neufs et d'une hyperplasie intimale qui peut conduire à une obstruction de l'artère et à une resténose due à la présence de l'endocalibreur.

L'implantation d'endocalibreurs coronaires joue un rôle de plus en plus important pour le traitement des cardiopathies coronaires chroniques, mais jusqu'à présent ces endocalibreurs n'ont pu résoudre le problème de la resténose et les taux de resténose après implantation de l'endocalibreur sont encore de 22 à 32 %. Comme on vient de le voir, cette resténose est le résultat d'une prolifération tissulaire néo-intimale qu'il conviendrait d'empêcher.

Aussi, il serait d'un grand intérêt de disposer de prothèses, notamment d'endocalibreurs coronaires, qui permettraient d'éviter cette prolifération tissulaire.

On a déjà envisagé de traiter des prothèses pour les charger de substances bioactives leur conférant des propriétés antithrombotiques et antimicrobiennes.

Ainsi, le document EP-A-596 615 [1] décrit des prothèses dont le substrat est en polymère qui sont recouvertes d'une couche de polymère greffé retenant un produit actif.

De telles prothèses conviennent mal comme endocalibreurs car le substrat polymère ne présente pas les caractéristiques mécaniques voulues.

Dans le cas de prothèses à substrats métalliques, la fixation sûre et durable d'un produit actif sur la prothèse est plus difficile à mettre en oeuvre.

Le document EP-A-0 873 732 [2] décrit un encalibreur sur lequel est fixé de l'héparine, par l'intermédiaire d'un revêtement comportant des groupes fonctionnels attirant l'héparine obtenu par exemple au moyen d'un plasma de méthane et d'un plasma de gaz ammoniac ou de monomère du type amine.

Le document US-A-4 879 135 [3] décrit des prothèses sur lesquelles on fixe un médicament, par l'intermédiaire d'un agent tensioactif anionique.

Le document EP-A-0 832 618 [4] décrit des stents ayant une anti-thrombogénicité à long terme, qui comportent un support métallique sur lequel est fixé par exemple de l'héparine au moyen d'un agent de couplage et d'un agent de réticulation, après oxydation de la surface métallique.

Ainsi, aucune de ces techniques ne permet de lier le revêtement portant la substance active au substrat métallique par une liaison covalente durable et sûre.

Ont été, par ailleurs décrits dans FR-A-2 187 849 [11] des matériaux biocompatibles et biofonctionnels d'applications biomédicales variées, dans lesquels on unit chimiquement une molécule biologique à un polymère ou un copolymère qu'on a préalablement greffé par rayonnement à un substrat polymère inerte.

La présente invention a précisément pour objet de nouvelles prothèses qui ne donnent pas lieu à cet inconvénient.

De plus, aucune des approches décrites jusqu'ici ne mentionne l'utilisation de substances bioactives greffées ayant des propriétés anti-resténose, alors que la resténose constitue l'une des réponses locales indésirables majeures lors de la pose d'endocalibreurs. L'objet de la présente invention est ainsi également de conférer aux revêtements la possibilité d'empêcher la resténose.

### Exposé de l'invention

Ainsi, l'invention a pour objet une prothèse bioactive comprenant un support dont la surface est en un matériau choisi parmi les métaux, les alliages métalliques, les matériaux semi-conducteurs, les polymères conducteurs, les fibres de carbone et leurs mélanges, ladite surface étant revêtue d'une couche de polymère ou copolymère à fonctions réactives greffé par liaison covalente sur la surface du support, et une substance bioactive fixée sur la prothèse par l'intermédiaire desdites fonctions réactives de façon à pouvoir être libérée progressivement sur un site d'implantation de la prothèse.

Cette prothèse présente ainsi une propriété supplémentaire due à la présence d'une substance bioactive appropriée, qui permet de traiter ou d'empêcher, directement sur le site d'implantation de la prothèse, le développement de phénomènes indésirables.

Selon l'invention, la surface du support de la prothèse est en un matériau qui peut être un métal, un alliage métallique, un polymère conducteur, un matériau semi-conducteur, des fibres de carbone, ou leurs mélanges.

Le support peut être réalisé en totalité en l'un de ces matériaux ou bien comporter une âme en n'importe quel type de matériau entourée d'une couche de l'un des matériaux définis ci-dessus.

Les métaux et alliages métalliques sont choisis, par exemple, parmi les aciers inoxydables, les alliages à base de cobalt ou de titane, les alliages à mémoire de forme, les métaux nobles comme l'or ou le platine, et les alliages de métaux nobles.

Les polymères conducteurs utilisables dans l'invention pour former la surface du support peuvent être de divers types. A titre d'exemples, on peut citer les polymères conducteurs formés à partir de monomères tels que le pyrrole, le thiophène, l'aniline ou leurs dérivés fonctionnalisés ou non, ainsi que les copolymèmes des monomères mentionnés ci-dessus.

Le matériau utilisé pour le support de la prothèse est choisi principalement en fonction des propriétés mécaniques que la prothèse doit présenter.

Dans le cas des endocalibreurs destinés à la cardiologie, on utilise avantageusement un support métallique, par exemple en acier inoxydable ou en alliage métallique à mémoire de forme.

Selon l'invention, la surface du support de la prothèse est revêtue d'une couche de polymère ou copolymère à fonctions réactives qui ont pour but de retenir de façon provisoire une substance bioactive qui sera libérée ensuite sur le site d'implantation de la prothèse.

Dans la suite du présent texte le terme polymère signifie non seulement un polymère à base de monomères identiques mais également un copolymère à base de monomères différents.

Les fonctions réactives appropriées pour cette fixation peuvent être choisies, par exemple, parmi les fonctions acide, ester, amide, amine et hydroxyle.

Le polymère utilisé pour former cette couche doit être biocompatible. On peut utiliser en particulier les polymères et/ou copolymères à monomère vinylique, fonctionnalisé ou non, le dextrane, les polymères conducteurs ainsi que tout polymère ou copolymère formé à partir de mélange de monomères vinyliques et/ou de monomères précurseurs de polymères conducteurs, fonctionnalisés ou non. A titre d'exemple de tels polymères, on peut citer les polymères acryliques et méthacryliques tels que le polyméthacrylate de méthyle, le polyméthacrylate d'éthyle, l'acide polyacrylique, l'acide polyméthacrylique, le poly(méthacrylate d'hydroxyéthyle) et le polyacrylamide, l'alcool polyvinylique, la poly(4-vinylpyridine), le dextrane, le polystyrène, les polymères conducteurs à base de thiophène, d'aniline, de pyrrole, ainsi que tout polymère ou copolymère utilisant des monomères obtenus par fonctionnalisation des monomères sus-cités. De tels polymères sont ou peuvent être munis de fonctions réactives appropriées, choisies en fonction du mode de fixation de la substance biologique.

Selon l'invention, la substance bioactive peut être fixée directement sur les fonctions réactives de la couche de polymère par une liaison covalente avec interposition éventuelle d'un bras espaceur entre les fonctions réactives et la substance.

La substance bioactive peut aussi être fixée sur les fonctions réactives de la couche de polymère par l'intermédiaire de microréservoirs contenant cette substance, qui sont fixés sur les fonctions réactives de la couche.

Ces microréservoirs peuvent comprendre une membrane externe microporeuse qui est liée aux fonctions réactives de la couche de polymère.

A titre d'exemple, cette membrane microporeuse peut être en polyacrylamide.

On peut aussi utiliser comme microréservoirs des billes d'agarose directement chargées de la substance bioactive ou chargées de sphérulites elles-mêmes chargées de la substance bioactive.

La substance bioactive utilisée est choisie en fonction des propriétés que l'on veut conférer à la prothèse. Cette substance bioactive peut appartenir par exemple au groupe des composés antimitotiques, anti-agrégants, anti-inflammatoires, antiresténose, antithrombotiques, immunosuppresseurs, anti-rejet et antibiotiques. On peut aussi utiliser comme substance bioactive un oligonucléotide anti-sens.

En effet, de tels oligonucléotides peuvent jouer le rôle de leurre pour l'ARNm et constituer des cibles pour des gènes de prolifération des cellules à divers niveaux. Dans le cas des prothèses cardiaques, ils peuvent s'opposer à la prolifération des cellules du muscle lisse.

Dans le cas de prothèses bioactives destinées à la cardiologie, la substance bioactive peut être choisie en particulier parmi la cyclosporine, la rapamycine, l'aspirine, la ticlopidine, la 3-déazaadénosine et le MCP-1.

Le MCP-1 est une protéine chimiotactique pour les macrophages.

La cyclosporine est un agent immunosuppresseur qui inhibe l'expression du facteur tissulaire dans les monocytes stimulés et les cellules du muscle lisse après implantation d'endocalibreurs coronaires.

La rapamycine est également un agent immunosuppresseur qui inhibe les kinases dépendantes de la cycline, et est utilisable pour empêcher la resténose des vaisseaux sanguins.

Selon l'invention, la couche de polymère peut servir de plus à retenir un composé héparinique par une liaison covalente, composé qui lui confère des propriétés anticoagulantes, antithrombotiques et anti-resténose.

L'invention a également pour objet un procédé de fabrication d'une prothèse bioactive telle que définie ci-dessus. Ce procédé comprend les étapes suivantes :
- 1) recouvrir un support dont la surface est en un matériau choisi parmi les métaux, les alliages métalliques, les matériaux semi-conducteurs, les polymères conducteurs, les fibres de carbone et leurs mélanges, avec un polymère à fonctions réactives, ledit polymère étant déposé directement sur la surface du support par électropolymérisation ou sur un polymère lui-même déposé sur la surface du support par électropolymérisation, et
- 2) fixer une substance bioactive sur la couche de polymère par l'intermédiaire des fonctions réactives de façon telle que cette substance puisse être libérée ensuite progressivement sur le site d'implantation de la prothèse.

Selon un premier mode de réalisation de la première étape du procédé de l'invention, on recouvre la surface du support d'un polymère, et on modifie ensuite le polymère pour introduire les fonctions réactives.

Selon un second mode de réalisation de cette première étape du procédé de l'invention, on dépose directement sur la surface du support le polymère à fonctions réactives.

Le dépôt de la couche de polymère est effectué par électropolymérisation en utilisant la surface du support comme électrode de dépôt.

L'électropolymérisation consiste en la polymérisation d'un monomère initiée. électrochimiquement par transfert électronique de la surface de l'électrode vers une molécule de monomère ou l'inverse. Elle donne lieu au dépôt dé deux types de polymères sur la surface de l'électrode, de structures chimiques très voisines et pourtant facilement séparables, comme il est décrit dans : P. Viel et al., Journal of Electroanalytical Chemistry, 470, 14 (1999) [5], dans J. Charlier et al., Journal of Electroanalytical Chemistry 465, 200 (1999) [6], et dans : C. Bureau et al., Journal of Adhesion, 58, 101, 1996 [7]. Ces deux types de polymères sont un polymère greffé chimiquement sur la surface de l'électrode, d'une épaisseur comprise entre 2 et 100 nm environ ; et un polymère non greffé, dont l'épaisseur peut aller jusqu'à 40 µm. Le polymère non greffé peut être éliminé par simple rinçage avec un solvant adéquat, alors que le polymère greffé résiste à des rinçages sous ultra-sons. Le mécanisme de formation des polymères aujourd'hui admis pour les réactions d'électropolymérisation cathodique est celui d'une propagation anionique, soit à partir de la surface (polymère greffé) soit directement dans la solution (polymère en solution), les deux mécanismes étant reliés par une bifurcation, comme il est décrit dans les références ci-dessus et dans : C. Bureau et al. Macromolecules, 30, 333 (1997) [8] (cf également les figures 1 et 2 de [8]). Un mécanisme analogue de type cationique, est cependant préconisé pour la polymérisation anodique de monomères vinyliques, comme décrit dans la référence [6]. Moyennant l'inclusion d'agents réticulants, le polymère non-greffé et le polymère greffé peuvent être solidarisés, et l'épaisseur du dépôt pilotée de 2 nm à 40 µm. Ce procédé de dépôt permet l'obtention d'un revêtement polymère sur la surface conductrice très solidement greffé par liaison covalente. Des fonctions réactives peuvent être implantées sur ce polymère greffé, soit en utilisant des monomères possédant déjà des fonctions réactives intéressantes (groupements esters), soit en réalisant la copolymérisation de ces monomères avec des monomères fonctionnalisés par des groupements fonctionnels éventuellement protégés, soit en provoquant l'apparition de ces groupements fonctionnels par un traitement chimique, électrochimique et/ou radiochimique ultérieur.

Lorsque le polymère peut être obtenu à partir d'un monomère polymérisable par voie anionique, choisi par exemple parmi l'acrylonitrile, le méthacrylonitrile, la 4-vinyl pyridine, le 4-chlorostyrène, le méthacrylate de méthyle, le méthacrylate d'éthyle, leur dérivés fonctionnalisés, les monomères à groupe époxy ainsi que les copolymères obtenus à partir de ces monomères, il s'agit d'une polymérisation anionique par électro-initiation cathodique.

Lorsque le polymère peut être obtenu à partir d'un monomère polymérisable par voie cationique, choisi par exemple parmi la N-vinyl pyrrolidone, la 4-vinyl pyridine, le pyrrole, le thiophène, l'aniline, ou tout monomère obtenu par fonctionnalisation de ces monomères de base, ou tout copolymère impliquant ces monomères, il s'agit d'une polymérisation cationique par électroinitiation anodique.

Selon l'invention, le polymère déposé par électropolymérisation peut constituer le polymère à fonctions réactives, ou il peut servir de couche intermédiaire sur laquelle on dépose ensuite le polymère à fonctions réactives.

Dans ce dernier cas, on dépose ensuite sur le polymère formé par électropolymérisation, le polymère à fonctions réactives par radiogreffage ou polymérisation par plasma d'un monomère précurseur de ce polymère.

Ce second dépôt peut être effectué, soit par radiogreffage, soit par polymérisation par plasma d'un monomère précurseur du polymère à fonctions réactives.

Le radiogreffage consiste à former, sous l'action de radiations ionisantes, des sites réactifs sur le polymère formé par électropolymérisation, à partir desquels on peut initier la polymérisation des monomères. Ces sites sont créés par irradiation, par exemple au moyen d'électrons, de rayons X, de rayons gamma ou de faisceaux d'ions lourds accélérés. L'irradiation peut être effectuée avant de mettre le polymère formé par électropolymérisation sur le support en contact avec le monomère ou simultanément à cette mise en contact.

On peut encore créer des sites de polymérisation du monomère sur le polymère formé par électropolymérisation, en le soumettant à l'action d'un plasma.

Les conditions de polymérisation sont choisies de façon à obtenir une couche de polymère d'épaisseur appropriée sur la couche intermédiaire formée par électropolymérisation sur le support de la prothèse.

Lorsqu'on introduit des fonctions réactives sur le polymère après dépôt de la couche sur le support, ceci peut être effectué par des procédés chimiques classiques de fonctionnalisation du polymère par les fonctions réactives appropriées. Par exemple, dans le cas du polystyrène, on peut introduire les fonctions réactives par fixation d'acide chlorosulfonique que l'on transforme ensuite pour introduire les fonctions réactives voulues.

Selon l'invention, on peut, si nécessaire, fixer de plus sur la couche de polymère un composé héparinique tel que l'héparine. Ceci peut être effectué lors du radiogreffage du monomère précurseur du polymère en ajoutant de l'héparine au milieu de greffage contenant le monomère précurseur à greffer. On peut utiliser dans ce but l'acide acrylique comme monomère précurseur, pour fixer l'héparine, comme il est décrit par Baquey et al, dans « Innov. tech. Biol. Med., vol. 2, n°4, 1981, page 378-389 » [9].

Dans la dernière étape du procédé de l'invention, on utilise les fonctions réactives de la couche de polymère pour fixer une substance bioactive de façon telle que celle-ci puisse être libérée ensuite sur le site d'implantation de la prothèse.

Pour réaliser cette fixation, on peut faire appel à des microréservoirs ou microcapsules fixés sur les fonctions réactives de la couche de polymère, ces microréservoirs étant remplis de la substance bioactive et dotés d'une paroi permettant de libérer cette substance sur une durée prolongée. On peut, par exemple, fixer les microréservoirs sur la couche de polymère par l'intermédiaire de fonctions associées à une membrane microporeuse entourant les microréservoirs, ladite membrane étant liée de façon covalente aux fonctions réactives de la couche de polymère.

Ces microréservoirs peuvent être des microbilles d'agarose directement chargées de la substance bioactive, sous forme de prodrogue polymère ou de sphérulites, elles-mêmes chargées de la substance bioactive. Les microbilles d'agarose sont entourées d'une membrane de microfiltration, par exemple en polyacrylamide, qui présente une microporosité telle qu'elle permet le passage de la substance active sur une durée prolongée.

Des microbilles d'agarose peuvent être formées de la façon suivante.

Tout d'abord, on prépare des billes d'agarose chargées de la substance bioactive ou de sphérulites par extrusion en veine huileuse, et on introduit les billes dans un capillaire où elles sont entraînées par un flux d'huile, puis enrobées par une solution photopolymérisable apportée par un capillaire adjacent. La solution photopolymérisable peut être par exemple à base d'acrylamide et de bis-acrylamide, et on peut former ainsi une membrane de polyacrylamide autour des billes. La porosité de cette membrane peut être contrôlée en réglant la concentration en bis-acrylamide de la solution.

On fixe ensuite les microbilles sur la couche de polymère qui, dans ce cas, peut comporter des fonctions réactives de type COOH pour former des liaisons amide entre ces groupes COOH et des groupes amine rapportés sur la membrane de polyacrylamide des microbilles. Ces groupes amine peuvent être formés à partir d'hexaméthylène diamine que l'on fixe sur le polyacrylamide par la technique de couplage d'hydrazine.

Cette technique de couplage à un polymère greffé en surface par de l'acide acrylique, de microbilles non chargées de substance active est décrite par Degert et al, Biomat, Art. Cells & Immob. Biotech, 21(4), 1993, pages 553-561 [10].

Dans le cas des sphérulites, il s'agit de complexes lipidiques constitués de couches emboîtées au sein desquelles ou entre lesquelles est incorporée la substance bioactive. Dans l'organisme, la libération de cette substance est obtenue par l'action des lipases endogènes. La fixation des sphérulites sur la couche de polymère peut être effectuée également au moyen de polyacrylamide en mettant les sphérulites en suspension dans une solution d'agarose extrudable et gélifiable en veine huileuse sous forme de billes qui sont circonscrites ultérieurement par une membrane de polyacrylamide à perméabilité contrôlable.

Lorsque la substance bioactive est une molécule simple telle que l'aspirine, on peut coupler chimiquement celle-ci sur les fonctions réactives de la couche de polymère par formation d'un composé capable de régénérer la substance bioactive sur le site d'implantation de la prothèse.

Dans le cas de l'aspirine (acide acétylsalicylique), on peut utiliser comme fonctions réactives, des fonctions hydroxyle pour former l'ester correspondant, soit l'acétylsalicylate.

Sur le site d'implantation de la prothèse, la substance bioactive pourra être régénérée et libérée par hydrolyse de l'acétylsalicylate.

Le polymère à fonctions hydroxyles peut être par exemple le poly(méthacrylate d'hydroxyéthyle), le dextrane ou l'alcool polyvinylique.

La fixation de la substance bioactive sur le polymère peut être effectuée après dépôt de la couche de polymère sur le support ou simultanément à ce dépôt.

Dans ce dernier cas, on effectue préalablement le couplage chimique de la substance bioactive à un monomère précurseur du polymère destiné à former la couche de polymère, et on forme la couche de polymère sur laquelle est fixée la substance bioactive par polymérisation du monomère couplé chimiquement à la substance bioactive ou par copolymérisation dudit monomère avec un monomère non fonctionnalisé.

Dans le cas de l'acide acétylsalicylique fixé sur du poly(méthacrylate d'hydroxyéthyle), on prépare tout d'abord l'acétyl salicylate d'hydroxyéthyl méthacrylate que l'on greffe ensuite sur le support, par exemple par radiogreffage.

D'autres caractéristiques et avantages de l'invention apparaîtront mieux à la lecture de la description qui suit, donnée bien entendu à titre illustratif et non limitatif.

### Exposé détaillé des modes de réalisation

### Exemple 1

Cet exemple illustre la réalisation d'endocalibreurs ou « stents », comprenant un support métallique recouvert de poly(méthacrylate de méthyle) (PMMA) sur lequel sont fixés des sphérulites préparées par la société CAPSULIS, qui sont chargées d'antibiotique.

On part d'un support en acier inoxydable (316 L) et on réalise un revêtement de polyméthacrylate de méthyle sur ce support par électropolymérisation. Dans ce but, on utilise une cellule électrolytique équipée de trois électrodes qui sont respectivement une électrode de travail, une électrode de référence et une électrode auxiliaire, le système étant commandé par un potentiostat connecté à un ordinateur. L'électrode de travail est constituée par l'endocalibreur métallique et on utilise une solution électrochimique comprenant le monomère, méthacrylate de méthyle (MMA), dissous à raison de 30 % en volume dans un solvant compatible constitué par du diméthyl sulfoxyde auquel on a ajouté un sel support électrolytique constitué par du perchlorate de tétrabutyl ammonium ou du tétrafluoroborate de tétrabutyl ammonium, à une concentration de 3.10⁻² mol/l. On dispose l'endocalibreur comme électrode de travail dans la solution électrolytique et on effectue le dépôt à un potentiel de -2,6 Volt (Ag+/Ag), c'est-à-dire par rapport à une électrode d'argent comme électrode de référence, avec une durée de polarisation de 100 ms. L'épaisseur de film déposé est d'environ 20 nm. Après lavage dans de l'éthanol et de la méthyl isobutylcétone, on vérifie par spectroscopie d'électrons émis sous l'action de rayons X et par spectrométrie infra-rouge (réflexion-absorption) que ce film est bien constitué de polyméthacrylate de méthyle.

Dans ces conditions, le mécanisme de polymérisation est un mécanisme anionique qui a lieu en solution et en surface.

Le polymère qui se forme en solution est éliminé par lavage, tandis que le polymère en surface est chimiquement greffé sur la surface S de l'électrode.

On fixe ensuite sur cette couche de polymère des sphérulites chargés d'un antibiotique en opérant de la façon suivante.

Tout d'abord, on prépare des sphérulites de 50 nm de diamètre chargées de 12,5 mg d'antibiotique par g de sphérulites.

Les sphérulites chargées d'antibiotique sont ensuite mises en suspension, à une concentration de 15 % en volume, dans une solution photopolymérisable comprenant de 15 à 20 % en poids d'acrylamide et 5 % en poids de bis-acrylamide, le reste étant constitué par le solvant, l'eau en l'occurrence contenant 10⁻⁵M de riboplasmine (photo-amorceur) et 10⁻³M de N,N'-tétraméthylène éthylène diamine TEMED (réducteurs).

On réalise l'association de billes polyacrylamide sur le support revêtu de polyméthacrylate de méthyle, après avoir hydrolysé le polyméthacrylate pour qu'il comporte des fonctions réactives COOH. On forme ainsi des liaisons covalentes entre les groupes amine apportés sur le polyacrylamide par couplage avec l'hexaméthylène diamine, et l'acide polyméthacrylique résultant de l'hydrolyse du PMMA, ce qui assure la fixation des sphérulites et de la substance antibiotique sur la couche de polymère.

### Exemple 2

Dans cet exemple, on recouvre un endocalibreur métallique de poly(4-vinylpyridine) sur laquelle on fixe de l'héparine. Pour réaliser le dépôt du polymère, on part d'une solution contenant 5 mol/l de 4-vinylpyridine et 5.10⁻² mol/l de perchlorate de tétraéthylammonium dans de l'acétonitrile.

On utilise une cellule électrochimique à trois électrodes comme précédemment, dans laquelle l'électrode de travail est constitué par l'endocalibreur en acier inoxydable (316L). On réalise le dépôt par électropolymérisation à un potentiel de -2,8 Volt (Ag+/Ag), pendant une durée de 40 s.

On obtient ainsi un film de poly(4-vinylpyridine) d'une épaisseur de 100 nanomètres. On modifie ensuite ce film en fixant dessus de l'héparine. Dans ce but, on quaternise tout d'abord la poly(4-vinylpyridine) par réaction avec un acide ou un chlorure d'acide, et on met ensuite à incuber dans une solution aqueuse d'héparinate de sodium (activité spécifique égale à ou voisine de 160 UI/mg) contenant une masse d'héparine proportionnelle à l'aire S du matériel à revêtir à raison de 10 µg d'héparine/cm² (M est donnée en µg par la formule suivante où S est en cm² : M = 100 S µg.) .

Après héparinisation du support revêtu de poly(4-vinylpyridine), on peut fixer sur la couche de poly(4-vinylpyridine) des micro ou des nanoréservoirs chargés de principe actif.

### Exemple 3

Dans cet exemple, on part d'endocalibreurs métalliques revêtus de PMMA comme dans l'exemple 1, et on dépose sur ce revêtement de l'acide polyacrylique par greffage après irradiation plasma.

Dans ce but, on expose les endocalibreurs revêtus de PMMA à un plasma créé par une RFGD (radiofrequency glow discharge) dans une atmosphère raréfiée Argon/Oxygène.

On opère dans les conditions suivantes :
Mélange Ar/02 : 50/50
Pression < 10 mmHg
Radiofréquence : 13,45 MHz
Puissance < 100 W.

Après irradiation, on dispose les endocalibreurs dans un récipient de réaction contenant une solution aqueuse d'acide acrylique à 20 % (v/v). Puis on place le récipient dans une enceinte thermostatée à une température de 65° pendant 5 heures.

On extrait alors les endocalibreurs métalliques revêtus de PMMA sur lequel est greffé l'acide polyacrylique dont la quantité est évaluée par titrage des fonctions -COOH (quelques 10⁻⁹ moles par mm²).

On fixe ensuite sur la couche d'acide polyacrylique des sphérulites chargées de principe actif en opérant comme dans l'exemple 1.

### Exemple 4

Cet exemple illustre la réalisation d'un endocalibreur métallique recouvert de PMMA comme dans l'exemple 1 et d'une couche de poly(méthacrylate d'hydroxyéthyle) sur laquelle est fixé de l'aspirine (acide acétyl salicylique).

On prépare tout d'abord de l'acétylsalicylate d'hydroxyéthyl méthacrylate par estérification du méthacrylate d'hydroxyéthyle par l'acide acétylsalicylique.

On réalise ensuite le greffage de cet acétylsalicylate sur l'endocalibreur recouvert de PTFE comme dans l'exemple 3 en remplaçant une partie de l'acide acrylique par l'acétylsalicylate d'hydroxyéthyl méthacrylate.

On obtient ainsi un endocalibreur sur lequel est fixé de l'aspirine, qui pourra être ensuite libéré par hydrolyse sur le site d'implantation de l'endocalibreur.

### Références citées

[1] : EP-A-596 615
[2] : EP-A-0 873 732
[3] : US-A-4 879 135
[4] : EP-A-0 832 618
[5] : P. Viel et al., Journal of Electroanalytical Chemistry, 470, 14 (1999).
[6] : J.Charlier et al., Journal of Electroanalytical Chemistry 465, 200 (1999).
[7] : C. Bureau et al., Journal of Adhesion, 58, 101, 1996.
[8] : C. Bureau et al. Macromolécules, 30, 333 (1997).
[9] : Baquey et al, Innov. tech. Biol. Med., vol. 2, n°4, 1981, pages 378-389
[10] : Degert et al, Biomat, Art. Cells & Immob. Biotech, 21(4), 1993, pages 553-561.
[11] : FR-A 2 187 849

## Revendications

1. Prothèse bioactive comprenant un support dont la surface est en un matériau choisi parmi les métaux, les alliages métalliques, les matériaux semi-conducteurs, les polymères conducteurs, les fibres de carbone et leurs mélanges, ladite surface étant revêtue d'une couche de polymère ou copolymère à fonctions réactives greffé par liaison covalente sur la surface du support, et une substance bioactive fixée sur la prothèse par l'intermédiaire desdites fonctions réactives de façon à pouvoir être libérée progressivement sur un site d'implantation de la prothèse.

2. Prothèse selon la revendication 1, dans laquelle la surface du support est en acier inoxydable, en alliage de cobalt ou de titane, en métal noble, en alliage de métal noble ou en alliage à mémoire de forme.

3. Prothèse selon l'une quelconque des revendications 1 et 2, dans laquelle les fonctions réactives du polymère de la couche sont choisies parmi les fonctions acide, ester, amide, amine ou hydroxyle.

4. Prothèse selon l'une quelconque des revendications 1 à 3, dans laquelle le polymère ou copolymère à fonctions réactives est choisi parmi les polymères et/ou copolymères à monomère vinylique, fonctionnalisé ou non, le dextrane, les polymères conducteurs ainsi que tout polymère ou copolymère formé à partir de mélange de monomères vinyliques et/ou de monomères précurseurs de polymères conducteurs, fonctionnalisés ou non.

5. Prothèse bioactive selon l'une quelconque des revendications 1 à 4, dans laquelle la substance bioactive est fixée sur la couche de polymère ou copolymère par l'intermédiaire de microréservoirs contenant cette substance fixés sur les fonctions réactives de la couche de polymère ou copolymère.

6. Prothèse bioactive selon la revendication 5, dans laquelle les microréservoirs comprennent une membrane externe microporeuse qui est liée aux fonctions réactives de la couche de polymère ou copolymère.

7. Prothèse selon la revendication 6, dans laquelle la membrane est en polyacrylamide.

8. Prothèse selon la revendication 5, dans laquelle les microréservoirs sont des billes d'agarose directement chargées de la substance bioactive ou chargées de sphérulites elles-mêmes chargées de la substance bioactive.

9. Prothèse selon l'une quelconque des revendications 1 à 8, dans laquelle la substance bioactive est choisie parmi les composés antimitotiques, anti-agrégants, anti-inflammatoires, anti-resténose, antithrombotiques, immunosuppresseurs, anti-rejet et antibiotiques, et les oligonucléotides anti-sens.

10. Prothèse selon la revendication 9, dans laquelle la substance bioactive est choisi parmi la cyclosporine, la rapamycine, l'aspirine, la ticlopidine, la 3-déazaadénosine et le MCP-1.

11. Prothèse selon l'une quelconque des revendications 1 à 10, dans laquelle la couche de polymère ou copolymère sert de plus à retenir un composé héparinique par une liaison covalente.

12. Procédé de fabrication d'une prothèse bioactive selon l'une quelconque des revendications 1 à 11, comprenant les étapes suivantes :
- 1) recouvrir un support dont la surface est en un matériau choisi parmi les métaux, les alliages métalliques, les matériaux semi-conducteurs, les polymères conducteurs, les fibres de carbone et leurs mélanges, avec un polymère ou copolymère à fonctions réactives, ledit polymère ou copolymère étant déposé directement sur la surface du support par électropolymérisation ou sur un polymère lui-même déposé sur la surface du support par électropolymérisation, et
- 2) fixer une substance bioactive sur la couche de polymère ou copolymère par l'intermédiaire des fonctions réactives de façon telle que cette substance puisse être libérée ensuite progressivement sur le site d'implantation de la prothèse.

13. Procédé selon la revendication 12, dans lequel, dans l'étape 1), on recouvre la surface du support d'un polymère ou copolymère, et on modifie ensuite le polymère ou copolymère pour introduire les fonctions réactives.

14. Procédé selon la revendication 12, dans lequel, dans l'étape 1, on dépose directement sur la surface du support le polymère ou copolymère à fonctions réactives.

15. Procédé selon la revendication 12, dans lequel on recouvre la surface du support d'un polymère par électropolymérisation et on dépose ensuite sur le polymère formé par électropolymérisation, le polymère ou copolymère à fonctions réactives par radiogreffage ou polymérisation par plasma d'un monomère précurseur de ce polymère.

16. Procédé selon l'une quelconque des revendications 12 à 15, dans lequel, on fixe de plus un composé héparinique sur la couche de polymère ou copolymère à fonctions réactives.

17. Procédé selon l'une quelconque des revendications 12 à 16, dans lequel la fixation de la substance bioactive sur les fonctions réactives de la couche de polymère ou copolymère consiste à coupler chimiquement ladite substance sur les fonctions réactives par formation d'un composé capable de régénérer la substance bioactive sur le site d'implantation de la prothèse

18. Procédé selon la revendication 17, dans lequel les fonctions réactives sont des fonctions hydroxyle et la substance bioactive est l'acide acétylsalicylique.

19. Procédé selon la revendication 18, dans lequel la couche de polymère à fonctions réactives est choisie parmi le poly(méthacrylate d'hydroxyéthyle), le dextrane et l'alcool polyvinylique.

20. Procédé selon l'une quelconque des revendications 17 à 19, dans lequel, on effectue préalablement le couplage chimique de la substance bioactive à un monomère précurseur du polymère ou copolymère destiné à former la couche de polymère, et on forme la couche de polymère sur laquelle est fixée la substance bioactive par polymérisation du monomère couplé chimiquement à la substance bioactive ou par copolymérisation dudit monomère avec un monomère non fonctionnalisé.

21. Procédé selon l'une quelconque des revendications 12 à 16, dans lequel, on fixe la substance bioactive sur la couche de polymère ou copolymère par l'intermédiaire de microréservoirs contenant cette substance fixés sur les fonctions réactives de la couche de polymère ou copolymère.

22. Procédé selon la revendication 21, dans lequel, on fixe les microréservoirs sur la couche de polymère ou copolymère par l'intermédiaire de fonctions associées à une membrane microporeuse entourant les micro-réservoirs, ladite membrane étant liée de façon covalente aux fonctions réactives de la couche de polymère ou copolymère.

23. Procédé selon la revendication 22, dans lequel la membrane est en polyacrylamide.

24. Procédé selon l'une quelconque des revendications 21 et 22, dans lequel les micro-réservoirs sont des billes d'agarose directement chargées de la substance bioactive ou chargées de sphérulites elles-mêmes chargées de la substance bioactive.

## Patentansprüche

1. Bioaktive Prothese, die umfasst einen Träger, dessen Oberfläche aus einem Material, ausgewählt aus Metallen, Metalllegierungen, halbleitenden Materialien, elektrisch leitenden Polymeren, Kohlenstoff-Fasern und ihren Mischungen, hergestellt ist, wobei die genannte Oberfläche von einer Schicht aus einem Polymer oder Copolymer mit reaktionsfähigen Funktionen bedeckt ist, die durch kovalente Bindung auf die Oberfläche des Trägers aufgepfropft ist, und eine bioaktive Substanz, die mittels der genannten reaktionsfähigen Funktionen so an der Prothese fixiert ist, dass sie am Ort der Implantation der Prothese allmählich freigesetzt werden kann.

2. Prothese nach Anspruch 1, in der die Oberfläche des Trägers aus nicht rostendem Stahl, einer Kobaltlegierung oder Titan, einem Edelmetall, einer Edelmetalllegierung oder einer Legierung mit Formspeichereigenschaften besteht.

3. Prothese nach einem der Ansprüche 1 und 2, in der die reaktionsfähigen Funktionen des Polymers der Schicht unter Säure-, Ester-, Amid-, Amin- oder Hydroxylfunktionen ausgewählt sind.

4. Prothese nach einem der Ansprüche 1 bis 3, in der das Polymer oder Copolymer mit reaktionsfähigen Funktionen ausgewählt ist aus Polymeren und/oder Copolymeren mit einem funktionalisierten oder nicht-funktionalisierten Vinylmonomer, Dextran, elektrisch leitenden Polymeren sowie irgendeinem Polymer oder Copolymer, das aus einer Mischung von funktionalisierten oder nicht-funktionalisierten Vinylmonomeren und/oder Vorläufermonomeren von elektrisch leitenden Polymeren hergestellt ist.

5. Bioaktive Prothese nach einem der Ansprüche 1 bis 4, in der die bioaktive Substanz auf der Schicht aus dem Polymer oder Copolymer fixiert ist mittels Mikroreservoirs, welche diese Substanz enthalten, die auf den reaktionsfähigen Funktionen der Schicht aus dem Polymer- oder Copolymer fixiert sind.

6. Bioaktive Prothese nach Anspruch 5, in der die Mikroreservoirs eine mikroporöse äußere Membran umfassen, die an die reaktionsfähigen Funktionen der Schicht aus dem Polymer oder Copolymer gebunden ist.

7. Prothese nach Anspruch 6, in der die Membran aus Polyacrylamid besteht.

8. Prothese nach Anspruch 5, in der die Mikroreservoirs Agarose-Kügelchen sind, die direkt mit der bioaktiven Substanz beladen sind oder die mit Sphärolithen beladen sind, die ihrerseits mit der bioaktiven Substanz beladen sind.

9. Prothese nach einem der Ansprüche 1 bis 8, in der die bioaktive Substanz ausgewählt ist aus antimitotischen Verbindungen, Antiaggregations-Verbindungen, antiinflammatorischen Verbindungen, Antirestenose-Verbindungen, antithrombotischen Verbindungen, Immunsuppressoren, Antiabstoßungs-Verbindungen und Antibiotika und Antisense-Oligonucleotiden.

10. Prothese nach Anspruch 9, in der die bioaktive Substanz ausgewählt ist aus Cyclosporin, Rapamycin, Aspirin, Ticlopidin, 3-Deazaadenosin und MCP-1.

11. Prothese nach einem der Ansprüche 1 bis 10, in der die Schicht aus dem Polymer oder Copolymer darüber hinaus dazu dient, durch eine kovalente Bindung eine Heparin-Verbindung zurückzuhalten.

12. Verfahren zur Herstellung einer bioaktiven Prothese nach einem der Ansprüche 1 bis 11, das die folgenden Stufen umfasst:
- 1) Beschichten eines Trägers, dessen Oberfläche aus einem Material, ausgewählt aus Metallen, Metalllegierungen, halbleitenden Materialien, elektrisch leitenden Polymeren, Kohlenstoff-Fasern und ihren Mischungen, besteht, mit einem Polymer oder Copolymer mit reaktionsfähigen Funktionen, wobei das Polymer oder Copolymer abgeschieden worden ist direkt auf der Oberfläche des Trägers durch Elektropolymerisation oder auf einem Polymer, das seinerseits auf der Oberfläche des Trägers durch Elektropolymerisation abgeschieden worden ist, und
- 2) Fixieren einer bioaktiven Substanz an der Schicht aus dem Polymer oder Copolymer mittels der reaktionsfähigen Funktionen in der Weise, dass diese Substanz anschließend an der Stelle der Implantation der Prothese allmählich freigesetzt werden kann.

13. Verfahren nach Anspruch 12, bei dem in der Stufe (1) die Oberfläche des Trägers aus einem Polymer oder Copolymer beschichtet wird und anschließend das Polymer oder Copolymer modifiziert wird, um reaktionsfähige Funktionen einzuführen.

14. Verfahren nach Anspruch 12, bei dem man in der Stufe (1) das Polymer oder Copolymer mit reaktionsfähigen Funktionen direkt auf die Oberfläche des Trägers aufbringt.

15. Verfahren nach Anspruch 12, bei dem man die Oberfläche des Trägers mit einem Polymer durch Elektropolymerisation beschichtet und anschließend auf das durch Elektropolymerisation gebildete Polymer das Polymer oder Copolymer mit reaktionsfähigen Funktionen durch Strahlungs-Bepfropfung oder durch Plasma-Polymerisation eines Vorläufer-Monomers des Polymers aufbringt.

16. Verfahren nach einem der Ansprüche 12 bis 15, bei dem man darüber hinaus eine Heparin-Verbindung auf der Schicht aus dem Polymer oder Copolymer mit reaktionsfähigen Funktionen fixiert.

17. Verfahren nach einem der Ansprüche 12 bis 16, bei dem die Fixierung der bioaktiven Substanz auf den reaktionsfähigen Funktionen der Schicht aus dem Polymer oder Copolymer darin besteht, die genannte Substanz mit den reaktionsfähigen Funktionen chemisch zu koppeln durch Bildung einer Verbindung, die in der Lage ist, die bioaktive Substanz an der Stelle der Implantation der Prothese zu regenerieren.

18. Verfahren nach Anspruch 17, bei dem die reaktionsfähigen Funktionen Hydroxyl-Funktionen sind und die bioaktive Substanz die Acetylsalicylsäure ist.

19. Verfahren nach Anspruch 18, bei dem die Schicht aus dem Polymer mit reaktionsfähigen Funktionen ausgewählt wird aus der Gruppe Poly(hydroxyethylmethacrylat), Dextran und Polyvinylalkohol.

20. Verfahren nach einem der Ansprüche 17 bis 19, bei dem man die chemische Kopplung der bioaktiven Substanz an ein Vorläufer-Monomer des Polymers oder Copolymers, das für die Bildung der Polymerschicht bestimmt ist, vorher durchführt und die Polymerschicht, auf der die bioaktive Substanz fixiert wird, durch Polymerisation des Monomers, das an die bioaktive Substanz chemisch gekoppelt ist, oder durch Copolymerisation des Monomers mit einem nicht-funktionalisierten Monomer bildet.

21. Verfahren nach einem der Ansprüche 12 bis 16, bei dem man die bioaktive Substanz an der Schicht aus dem Polymer oder Copolymer fixiert mittels Mikroreservoirs, welche diese Substanz enthalten, die an den reaktionsfähigen Funktionen der Schicht aus dem Polymer oder Copolymer fixiert sind.

22. Verfahren nach Anspruch 21, bei dem man die Mikroreservoirs an der Schicht aus dem Polymer oder Copolymer fixiert mittels Funktionen, die mit einer mikroporösen Membran assoziiert sind, welche die Mikroreservoirs umgibt, wobei die genannte Membran auf kovalente Weise mit den reaktionsfähigen Funktionen der Schicht aus dem Polymer oder Copolymer verbunden ist.

23. Verfahren nach Anspruch 22, worin die Membran aus Polyacrylamid besteht.

24. Verfahren nach einem der Ansprüche 21 und 22, worin die Mikroreservoirs Kügelchen aus Agarose sind, die direkt mit der bioaktiven Substanz beladen sind, oder die mit Sphärolithen beladen sind, die ihrerseits mit der bioaktiven Substanz beladen sind.

## Claims

1. Bioactive implant comprising a substrate, wherein the surface is made of a material chosen from metals, metallic alloys, semiconductor materials, conductive polymers, carbon fibres and their mixtures, said surface being coated with a polymer or copolymer layer with reactive functions grafted by means of a covalent bond on the substrate surface and a bioactive substance fixed on the implant by means of said reactive functions to enable progressive release on an implant site.

2. Implant according to claim 1, wherein the substrate surface is made of stainless steel, cobalt or titanium-based alloy, noble metal, noble metal alloy or shape-retentive alloy.

3. Implant according to any of claims 1 and 2, wherein the reactive functions of the polymer of the layer are chosen from the acid, ester, amide, amine or hydroxyl functions.

4. Implant according to any of claims 1 to 3, wherein the reactive function polymer or copolymer is chosen from vinyl monomer polymers and/or copolymers, functionalized or not, dextran, conductive polymers and any polymer or copolymer formed from mixtures of vinyl monomers and/or conductive polymer precursor monomers, functionalized or not.

5. Bioactive implant according to any of claims 1 to 4, wherein the bioactive substance is fixed on the polymer or copolymer layer by means of microreservoirs containing said substance fixed on the reactive functions of the polymer or copolymer layer.

6. Bioactive implant according to claim 5, wherein the microreservoirs comprise a microporous outer membrane which is coupled with the reactive functions of the polymer or copolymer layer.

7. Implant according to claim 6, wherein the membrane is made of polyacrylamide.

8. Implant according to claim 5, wherein the microreservoirs are agar beads filled directly with the bioactive substance or filled with spherulites in turn filled with the bioactive substance.

9. Implant according to any of claims 1 to 8, wherein the bioactive substance is chosen from antimitotic, antiaggregation, antiinflammatory, antirestenotic, antithrombotic, immunosuppressive, antirejection and antibiotic compounds and reverse oligonucleotides.

10. Implant according to claim 9, wherein the bioactive substance is chosen from cyclosporin, rapamycin, aspirin, ticlopidine, 3-deazaadenosine and MCP-1.

11. Implant according to any of claims 1 to 10, wherein the polymer or copolymer layer is also used to hold a heparin compound with a covalent bond.

12. Method to produce a bioactive implant according to any of claims 1 to 11, comprising the following steps:
- 1) coat the substrate wherein the surface is made of a material chosen from metals, metallic alloys, semiconductor materials, conductive polymers, carbon fibres and their mixtures, with a polymer or copolymer with reactive functions, said polymer or copolymer being deposited directly on the surface of the substrate by electropolymerization or on a polymer which has itself been deposited on the surface of the substrate by electropolymerization, and
- 2) fix a bioactive substance on the polymer or copolymer by means of the reactive functions such that said substance can subsequently be released progressively on the implant site.

13. Method according to claim 12, wherein, in step 1), the surface of the substrate is coated with a polymer or copolymer and the polymer or copolymer is then modified to introduce the reactive functions.

14. Method according to claim 12, wherein, in step 1), the reactive function polymer or copolymer is deposited directly onto the surface of the substrate.

15. Method according to claim 12, wherein the surface of the substrate is covered with a polymer by electropolymerization and then on the polymer formed by electropolymerization is deposited the reactive function polymer or copolymer by radiografting or plasma polymerization of a precursor monomer of said polymer.

16. Method according to any of claims 12 to 15, wherein a heparin compound is also fixed onto the reactive function polymer or copolymer layer.

17. Method according to any of claims 12 to 16, wherein the fixation of the bioactive substance on the reactive functions of the polymer or copolymer layer consists of coupling said substance chemically onto the reactive functions by forming a compound capable of regenerating the bioactive substance on the implant site.

18. Method according to claim 17, wherein the reactive functions are hydroxyl functions and the bioactive substance is acetylsalicylic acid.

19. Method according to claim 18, wherein the reactive function polymer layer is chosen from poly(hydroxyethyl methacrylate), dextran and polyvinyl alcohol.

20. Method according to any of claims 17 to 19, wherein the chemical coupling of the bioactive substance with a precursor monomer of the polymer or copolymer intended to form the polymer layer is carried out beforehand and the polymer layer on which the bioactive substance is fixed is formed by polymerization of the monomer coupled chemically with the bioactive substance or by copolymerization of said monomer with a non-functionalized monomer.

21. Method according to any of claims 12 to 16, wherein the bioactive substance is fixed on the polymer or copolymer layer by means of microreservoirs containing said substance fixed on the reactive functions of the polymer or copolymer layer.

22. Method according to claim 21, wherein the microreservoirs are fixed onto the polymer or copolymer layer by means of functions associated with a microporous membrane surrounding the microreservoirs, said membrane being linked to the reactive functions of the polymer or copolymer layer via a covalent bond.

23. Method according to claim 22, wherein the membrane is made of polyacrylamide.

24. Method according to either of the claims 21 and 22, wherein the microreservoirs are agar beads filled directly with the bioactive substance or filled with spherulites in turn filled with the bioactive substance.
